# EUROPEAN PATENT SPECIFICATION

(11) **EP 3 391 893 B1**
(45) Date of publication and mention of the grant of the patent: **29.07.2020**
(21) Application number: 16874302.9
(22) Date of filing: 08.04.2016
(51) Int. Cl.: A61K 36/315, A61K 36/704, A61K 36/537, A61K 36/36, A61K 33/36, A61P 35/02

(54) **PHARMACEUTICAL COMPOSITION FOR TREATING LEUKEMIA AND PREPARATION METHOD THEREOF**
MEDIZINISCHE ZUSAMMENSETZUNG ZUR BEHANDLUNG VON LEUKÄMIE UND HERSTELLUNGSVERFAHREN DAFÜR
COMPOSITION MÉDICAMENTEUSE POUR TRAITER LA LEUCÉMIE ET SON PROCÉDÉ DE PRÉPARATION

(30) Priority: 17.12.2015 CN 201510955934
(43) Date of publication of application: 24.10.2018
(73) Proprietor: Yi Fan Pharmaceutical Academy (Beijing) Co., Ltd., Beijing 100000 (CN); Yifan Pharmaceutical Co., Ltd., Hangzhou Zhejiang 310000 (CN)
(72) Inventor: CHENG, Xianfeng, Hangzhou Zhejiang 310000 (CN); ZHOU, Wenyan, Hangzhou Zhejiang 310000 (CN); LIANG, Xiaodong, Beijing 100000 (CN); QIAN, Liping, Hangzhou Zhejiang 310000 (CN)
(74) Representative: Würmser, Julian
(86) International application number: PCT/CN2016/078911
(87) International publication number: WO 2017/101236

(56) References cited:
- CN-A- 1 615 970
- CN-A- 101 590 103
- CN-A- 105 362 340
- L. WANG ET AL: "Dissection of mechanisms of Chinese medicinal formula Realgar-Indigo naturalis as an effective treatment for promyelocytic leukemia", PROCEEDINGS OF THE NATIONAL ACADEMY OF SCIENCES OF THE UNITED STATES OF AMERICA, vol. 105, no. 12, 25 March 2008 (2008-03-25), pages 4826-4831, XP055546067, US ISSN: 0027-8424, DOI: 10.1073/pnas.0712365105
- YANFENG LIU ET AL: "Long-term outcome of 31 cases of refractory acute promyelocytic leukemia treated with compound realgar natural indigo tablets administered alternately with chemotherapy", ONCOLOGY LETTERS, vol. 10, no. 2, 2 June 2015 (2015-06-02), pages 1184-1190, XP055546097, GR ISSN: 1792-1074, DOI: 10.3892/ol.2015.3308
- HONG-HU ZHU ET AL: "Oral Tetra-Arsenic Tetra-Sulfide Formula Versus Intravenous Arsenic Trioxide As First-Line Treatment of Acute Promyelocytic Leukemia: A Multicenter Randomized Controlled Trial", JOURNAL OF CLINICAL ONCOLOGY, vol. 31, no. 33, 20 November 2013 (2013-11-20), pages 4215-4221, XP055546143, US ISSN: 0732-183X, DOI: 10.1200/JCO.2013.48.8312
- MA, BIN ET AL.: 'Advances of Toxicology of Realgar and Its Prescriptions in China' CHINESE ARCHIVES OF TRADITIONAL CHINESE MEDICINE vol. 31, no. 8, 31 August 2013, pages 1623 - 1625, XP009508429
- HAN, JIE ET AL.: 'Discussion on the safety of preparation containing realgar in 2005 edition of ' Chinese Pharmacopoeia' CHINA JOURNAL OF CHINESE MATERIA MEDICA vol. 33, no. 13, 31 July 2008, pages 1638 - 1640, XP009508428

## Description

### Technical field

The invention relates to the field of leukemia drugs, in particular to a pharmaceutical composition for treating leukemia and a preparation method thereof.

### Background

At present, over 190 kinds of compound preparations which contain realgar that have been formally included in the current four national standards for traditional Chinese medicines, including about 2.78% of all prescriptions. Long-term administration of traditional Chinese medicines containing excess realgar can easily cause chronic arsenic poisoning, and even death, so realgar should not be taken for a long time and overdose of realgar should be avoided. The daily intake of Realgar prescribed by the 2010 version 〈〈Chinese Pharmacopoeia〉〉 must not exceed 0.1 g. If the human body takes realgar in large doses for a long time, heavy metals such as arsenic may accumulate in the body, causing severe liver and kidney damage, and may damage the human blood system and nervous system. For the toxicity of realgar, most of the herbal works have recorded that the ancient people have already forbidden the use of large doses of single realgar medicine. According to the prior art, the prescription dosage of realgar is too large, generally the prescribed dosage is three times a day and three tablets for each time, that is, the minimum amount is 9 tablets per day. This realgar dosage exceeds the standard greatly, so the amount of realgar needs to be adjusted.

The patent application No. 200410050453.1 discloses an anti-leukemia traditional Chinese medicines preparation, compound realgar natural indigo tablets and preparation method thereof, the composition of the compound realgar natural indigo tablets includes realgar, and the main component of the realgar is the arsenic disulfide or the arsenic tetrasulfide. Realgar also contains impurities, the main impurity is arsenic trioxide (commonly known as arsenic), which is highly toxic, so realgar is toxic, and is included in the 28 kinds of toxic herbs in China, and is a relatively toxic traditional Chinese medicine. The existing compound realgar natural indigo tablets need to be taken in a relatively large dose to achieve the purpose of treating leukemia. However, due to the high toxicity of the compound realgar natural indigo tablets, large amounts of long-term use of the compound realgar natural indigo tablets may cause great harm to the liver and kidneys.

In addition, in the existing preparation process, the preparation of the extractum is often inconvenient, because the concentration is so large that the next process step cannot be performed.

At the same time, the existing compound realgar natural indigo tablets and retinoic acid (a drug used to treat leukemia, often used together with arsenical to treat leukemia) will have certain cross-resistance, which is not conducive to the treatment of leukemia.

Therefore, the present disclosure is specifically proposed.

### Summary

The first objective of the present disclosure is to provide a pharmaceutical composition for treating leukemia. The pharmaceutical composition greatly reduces the content of realgar and increases the dosage of the salvia miltiorrhiza on the basis of the existing compound realgar natural indigo tablet. After clinical verification, it was surprisingly found that the curative effect of the pharmaceutical composition in the present disclosure has been significantly enhanced and improved.

The second objective of the present disclosure is to provide a method for preparing a pharmaceutical composition for treating leukemia, based on the preparation method of the existing compound realgar natural indigo tablet, the method of the present disclosure improves the preparation method of salvia miltiorrhiza, the amount of water added to salvia miltiorrhiza is reduced, the number of decocting times is reduced, and the decocting time is shortened to ensure that the active ingredients of salvia miltiorrhiza are not damaged. The experimental results show that the active ingredients of salvia miltiorrhiza can be well maintained and the extraction rate is maintained at a high level.

Salvia miltiorrhiza has the functions of analgesia, promoting blood circulation and clearing the heart, and is involved in various pharmacological effects such as anti-myocardial ischemia, anti-cerebral ischemia, anti-thrombosis, improvement of microcirculation, promotion of tissue repair and regeneration, and sedation and analgesia. Tanshinone and tanshinol, which are the active ingredients of salvia miltiorrhiza, are important substances for pharmacological effects.

Studies have confirmed that tanshinone is the main antitumor active ingredient in salvia miltiorrhiza. It has long been reported that salvia miltiorrhiza can prolong the survival time of mouse with Ehrich ascites carcinoma and has a synergistic effect on the antitumor activity of camptothecin and cyclophosphamide. Later, it was reported that salvia miltiorrhiza also has a killing effect on Ehrich ascites carcinoma mouse. Tanshinones have a wide range of phenanthrenequinone structure that are the basis of their cytotoxicity. Among them, phenanthrene ring structures bind to DNA molecules, while furan ring and anthraquinone structures can generate free radicals to cause DNA damage and inhibit the synthesis of tumor cell DNA. The results show that after a non-toxic dose treatment of tanshinone IIA (0.5µg/ml) and all-*trans* retinoic acid (ATRA) (0.5µg/ml), the cell morphology tends to be benign differentiation, the growth of the cell has slowed down, the colony formation rate and tritium-labeled-thymidine(3H-TdR) incorporation rate are obviously reduced, the tumor formation time on nude mice prolongs and the tumorigenic ability is significantly reduced. After statistical analysis, both tanshinone IIA and ATRA have better induced differentiation effects on ME180 cells, and the difference between tanshinone IIA and ATRA is not significant (P>0.05,i.e., the probability is greater than 0.5). After treatment with a non-toxic dose of tanshinone IIA (0.5µg/ml) to the ME180 cell line, cell RNA dot blot hybridization is performed, the expression of c-myc and H-ras oncogenes are significantly reduced, it is speculated that the induced differentiation mechanism of tanshinone II A on ME180 cells may be the inhibition of oncogene expression related to cell proliferation. There is a balance between proliferation, differentiation, and apoptosis of normal cells, whereas tumor cells multiply indefinitely, differentiation is blocked, and apoptosis is suppressed. Current studies have found that many anticancer drugs exert anticancer effects by inhibiting the proliferation of tumor cells, inducing differentiation and/or apoptosis of tumor cells. The studies have found that a non-toxic dose of tanshinone IIA (0.5µg/ml) acting on HL-6 cells can induce differentiation and maturation of tumor cells, accompanied by apoptosis. Flow cytometry analysis has revealed that cells in S phase are significantly reduced, cells are arrested in the G0/G1 phase, cell proliferation index is decreased, apoptosis occurs in some cells, a subdiploid apoptotic peak appears, and the oncogene c-fos expression is increased, and c-myc and bcl-2 are decreased.

Therefore, the anti-tumor mechanism of tanshinone may induce tumor cell differentiation, induce apoptosis or induce differentiation and maturation of tumor cells, and eventually lead to apoptosis. Tanshinone IIA significantly inhibits the growth of SGC7901 human gastric adenocarcinoma cells in vitro and induces apoptosis. The possible mechanism is that tanshinone IIA can block cells in G0/G1 phase, making cells unable to enter S phase.

Hyperfibrinogenemia exacerbates tumor blood stasis syndrome, and the essence of tumor blood stasis syndrome is blood coagulation fibrinolytic platelet dysfunction. Tanshinone has the function of activating blood and removing blood stasis, which is throughout the treatment of tumors by traditional Chinese medicine.

In summary, tanshinone, the extract of salvia miltiorrhiza, not only has the effects of natural anti-oxidation, antibacterial, anti-inflammatory, and sex hormone, but also has significant effects on cardiovascular, cerebrovascular, and diabetes, more importantly, tanshinone has obvious anti-tumor effects and can be widely used in the treatment of clinical, surgery, gynecology, ophthalmology and otorhinolaryngology diseases and has achieved exact results. Tanshinone preparation is easy to use, safe and economical. Tanshinone preparations are taken orally and absorbed by the intestine and rapidly distributed throughout the body, and have the characteristics of strong effects, slow excretion, no resistance and side effects, and are worthy of wide application.

In order to achieve the above objectives of the present disclosure, the following technical solutions are specially adopted.

A pharmaceutical composition for treating leukemia, mainly made of the following raw materials by weight percentage, 2 to 8 percent of realgar, 25 to 42 percent of indigo naturalis, 50 to 60 percent of salvia miltiorrhiza, and 6 to 10 percent of radix pseudostellariae.

According to the prior art, the amount of salvia miltiorrhiza is too small, according to the 2010 edition 〈〈Chinese Pharmacopoeia〉〉, daily intake of salvia miltiorrhiza is 10-15 g. According to the normal dose, three times a day and three tablets each time, that is, 9 tablets per day, daily intake of salvia miltiorrhiza is less than half of the amount prescribed by the 〈〈Chinese Pharmacopoeia〉〉, and cannot reach the results of stasis-removing and blood-activating, and anti-tumor, so the amount of salvia miltiorrhiza needs to be adjusted. Therefore, based on the prior art, the present disclosure increases the amount of salvia miltiorrhiza from 36%- 46% to 50% - 60%, and reduces the amount of realgar from 12% - 18% to 2%-8%. By Clinical trials, it has been surprisingly found that pharmaceutical compositions modified according to the prior art have clinically treated leukemia with a complete remission rate of 92.9%, curative effect has been obviously increased and improved, and toxic side effects have been significantly reduced.

In order to enhance the synergistic effect among the raw materials, preferably, the raw materials of the pharmaceutical composition by weight percentage include, 3 to 5 percent of realgar, 27 to 35 percent of indigo naturalis, 52 to 57 percent of salvia miltiorrhiza, and 7 to 9 percent of radix pseudostellariae.

Preferably, the indigo naturalis is excellent grade indigo naturalis or special grade indigo naturalis. Based on the existing methods, the indigo naturalis is more preferably the special grade indigo naturalis, and the special grade indigo naturalis is formed after being cleaned , refined, and sterilized. The indirubin content of the special grade indigo naturalis is improved, and the concerted application of the minister drug is increased.

The present disclosure also provides a method for preparing a pharmaceutical composition for treating leukemia, including the following steps:
mixing a realgar, a radix pseudostellariae and an indigo naturalis powder uniformly to obtain a mixture for standby application;
adding water to the salvia miltiorrhiza in accordance with liquid-to-solid ratio of 8ml/1g-9ml/1g to obtain a solution, decocting the solution 1-2 times, and for 50-55min each time, then filtering the solution to obtain a filtrate, the filtrate is then concentrated to get a concentrate at a temperature of 48-54 ° C;
adding the mixture to the concentrate, and then mixing the mixture and the concentrate, making a corresponding pharmaceutical dosage form as required.

The active ingredients of salvia miltiorrhiza, such as tanshinone and salvianolic acid, are unstable under long-term high-heat conditions. The extraction time in the prior art is 1-2 hours. Under these conditions, most of the active ingredients of salvia miltiorrhiza have been destroyed. In order to ensure that the active ingredients of salvia miltiorrhiza are not destroyed, there is a strict requirement on the extraction time of salvia miltiorrhiza. The preparation method of pharmaceutical composition for treating leukemia provided by the present disclosure, based on the preparation method of the existing compound realgar natural indigo tablet, improves the preparation method of salvia miltiorrhiza, the amount of water added to salvia miltiorrhiza is reduced, the number of decocting times is reduced, and the decocting time is shortened to ensure that the active ingredients of salvia miltiorrhiza are not damaged. The experimental results show that the active ingredients of salvia miltiorrhiza are tanshinone IIA (C₁₉H₁₈O₃), tanshinone I (C₁₈H₁₂O₃), and tanshinone IIB (C₁₉H₁₈O₄), etc., all of which can be well extracted. Tanshinone IIA, the active ingredient as the control index component, is detected by high performance liquid chromatography and the extraction rate of tanshinone IIA reaches 75%. The activity of each active ingredient is well maintained and the extraction rate is maintained at a high level.

In order to better remove impurities, reduce toxicity, and obtain finer realgar powder, further, specific steps of preparing the realgar into a powder form are as follows: the realgar is selected, and a precipitate is taken after water grind, and then the precipitate is dried and grinded for later use.

The radix pseudostellariae is gradually broken to form powder. Further, specific steps of preparing the radix pseudostellariae into a powder form are as follows: pulverizing the radix pseudostellariae into a fine powder; then, the fine powder is crushed to a very fine powder for later use.

Among them, the fine powder refers to powder that can completely pass through the No. 6 sieve and no less than 95% can pass through the No. 7 sieve; the very fine powder refers to powder that can completely pass through the No. 8 sieve and no less than 95% can pass the No. 9 sieve.

Further, the pharmaceutical dosage form is tablets, pills, capsules, or granules.

Specifically, the pharmaceutical dosage form is tablets; the concentrate is a clear paste with a relative density of 1.15 to 1.20 measured at a temperature of 50°C; and then the clear paste is mixed with the mixture, granulated, dried, sized, compressed, and coated to obtain the tablets.

Further, the pharmaceutical dosage form is pills; the concentrate is concentrated to a thick paste, then the thick paste is dried under reduced pressure into a dry paste, the dry paste is crushed into a fine powder to obtain a dry fine powder;
the dry fine powder is mixed with the mixture, and placed in a pill making machine with water and then dried to obtain the pills.

Further, the pharmaceutical dosage form is capsules; the concentrate is a clear paste with a relative density of 1.15 to 1.20measured at a temperature of 50°C; and then the clear paste is mixed with the mixture, granulated, dried, sized, compressed, and filled into capsules.

In addition, the pharmaceutical dosage form of the pharmaceutical composition for treating leukemia provided by the present disclosure is not limited to the above-mentioned four kinds, and other dosage forms may also be prepared according to the methods of conventional pharmaceutical preparations.

The pharmaceutical composition for treating leukemia provided by the present disclosure is mainly clinically used for acute promyelocytic leukemia or chronic myelogenous leukemia.

Compared with the prior art, the beneficial effects of the present disclosure are as follows:
(1) The pharmaceutical composition for treating leukemia provided by the present disclosure increases the amount of salvia miltiorrhiza from 36% - 46% to 50% - 60%, and reduces the amount of realgar from 12%- 18% to 2% - 8%. Clinical trials have surprisingly found that pharmaceutical compositions modified according to the prior art are clinically used for the treatment of leukemia, and are mainly used for acute promyelocytic leukemia and chronic myelogenous leukemia, the curative effect has been significantly enhanced and improved, and the toxic and side effects have been greatly reduced, and the safety has been obviously guaranteed.
(2) The present disclosure further defines the proportion of raw materials so as to enhance the synergistic effect among the raw materials.
(3) The present disclosure also provides a preparation method of the pharmaceutical composition for treating leukemia, which improves the preparation method of the salvia miltiorrhiza, the amount of water added to salvia miltiorrhiza is reduced, the number of decocting times is reduced, and the decocting time is shortened to ensure that the active ingredients of salvia miltiorrhiza are not damaged. The experimental results show that the active ingredients of salvia miltiorrhiza can be well maintained and the extraction rate is maintained at a high level.
(4) The pharmaceutical composition for treating leukemia provided by the present disclosure is suitable for dosage forms including but not limited to tablets, capsules, granules, pills and the like.

### Detailed Description

The implementations of the present disclosure will be described in detail with the following embodiments. If no specific conditions are indicated in the examples, it shall be performed according to conventional conditions or conditions recommended by the manufacturer. The reagents or instruments used which are not specified by the manufacturer are all commercially available conventional products.

### Embodiment 1

Taking realgar, indigo naturalis, salvia miltiorrhiza and radix pseudostellariae as the components, according to the percentage by weight, realgar, indigo naturalis, salvia miltiorrhiza, and radix pseudostellariae were prepared respectively with the weight percentage of 2%, 42%, 50% and 6%.

The realgar is selected, and a precipitate is taken after the water grind, and then the precipitate is dried and grinded for later use.

The radix pseudostellariae is first pulverized into a fine powder; then, the fine powder is crushed to a very fine powder and sterilized for later use.

The special grade indigo naturalis is selected, cleaned, refined, dried and sterilized for later use.

The fine powders of the above three pharmaceutical compositions are mixed for later use.
adding water to the salvia miltiorrhiza in accordance with liquid-to-solid ratio of 8ml/1g to obtain a solution, decocting the solution 2 times, for 50 min each time, then filtering the solution to obtain a filtrate, the filtrate is then combined and concentrated to get a clear paste with a relative density of 1.15 to 1.24 (50° C) at a temperature of 48-54 ° C.

The homogenized fine powders of the above three pharmaceutical compositions were added to the clear paste, granulated, dried, sized, and compressed (weight 0.25 g), and coated to obtain the pharmaceutical composition for treating leukemia.

### Embodiment 2

Taking realgar, indigo naturalis, salvia miltiorrhiza and radix pseudostellariae as the components, according to the percentage by weight, realgar, indigo naturalis, salvia miltiorrhiza, and radix pseudostellariae were prepared respectively with the weight percentage of 8%, 25%, 60% and 7%.

The realgar is selected, and a precipitate is taken after the water grind, and then the precipitate is dried and grinded for later use.

The radix pseudostellariae is first pulverized into a fine powder; then, the fine powder is crushed to a very fine powder and sterilized for later use.

The special grade indigo naturalis is selected, cleaned, refined, dried and sterilized for later use.

The fine powders of the above three pharmaceutical compositions are mixed for later use.
adding water to the salvia miltiorrhiza in accordance with liquid-to-solid ratio of 9ml/1g to obtain a solution, decocting the solution 2 times, for 55 min each time, then filtering the solution to obtain a filtrate, the filtrate is then combined and concentrated to get a clear paste with a relative density of 1.15 to 1.24 (50° C) at a temperature of 48-54 ° C.

The homogenized fine powders of the above three pharmaceutical compositions were added to the clear paste, granulated, dried, sized, and compressed (weight 0.25 g), and coated to obtain the pharmaceutical composition for treating leukemia.

### Embodiment 3

Taking realgar, indigo naturalis, salvia miltiorrhiza and radix pseudostellariae as the components, according to the percentage by weight, realgar, indigo naturalis, salvia miltiorrhiza, and radix pseudostellariae were prepared respectively with the weight percentage of 5%, 27%, 58% and 10%.

The realgar is selected, and a precipitate is taken after the water grind, and then the precipitate is dried and grinded for later use.

The radix pseudostellariae is first pulverized into a fine powder; then, the fine powder is crushed to a very fine powder and sterilized for later use.

The special grade indigo naturalis is selected, cleaned, refined, dried and sterilized for later use.

The fine powders of the above three pharmaceutical compositions are mixed for later use.
adding water to the salvia miltiorrhiza in accordance with liquid-to-solid ratio of 8ml/1g to obtain a solution, decocting the solution 2 times, for 55 min each time, then filtering the solution to obtain a filtrate, the filtrate is then combined and concentrated to get a thick paste, then the thick paste is dried and crushed into a fine powder .

The fine powder of salvia miltiorrhiza is added into the homogenized fine powders of the above three pharmaceutical compositions, mixed uniformly, placed in a pill making machine, panned with water and dried to obtain pills with a weight of 0.25 g.

### Embodiment 4

Taking realgar, indigo naturalis, salvia miltiorrhiza and radix pseudostellariae as the components, according to the percentage by weight, realgar, indigo naturalis, salvia miltiorrhiza, and radix pseudostellariae were prepared respectively with the weight percentage of 4%, 35%, 52% and 9%.

The realgar is selected, and a precipitate is taken after the water grind, and then the precipitate is dried and grinded for later use.

The radix pseudostellariae is first pulverized into a fine powder; then, the fine powder is crushed to a very fine powder and sterilized for later use.

The special grade indigo naturalis is selected, cleaned, refined, dried and sterilized for later use.

The fine powders of the above three pharmaceutical compositions are mixed for later use.
adding water to the salvia miltiorrhiza in accordance with liquid-to-solid ratio of 9ml/1g to obtain a solution, decocting the solution 2 times, for 50 min each time, then filtering the solution to obtain a filtrate, the filtrate is then combined and concentrated to get a clear paste with a relative density of 1.15 to 1.24 (50° C) at a temperature of 48-54°C.

The homogenized fine powders of the above three pharmaceutical compositions were added to the clear paste, granulated, dried, sized, and filled into a capsule (the weight is 0.25 g) to obtain the pharmaceutical composition for treating leukemia in a capsule form.

### Embodiment 5

Taking realgar, indigo naturalis, salvia miltiorrhiza and radix pseudostellariae as the components, according to the percentage by weight, realgar, indigo naturalis, salvia miltiorrhiza, and radix pseudostellariae were prepared respectively with the weight percentage of 3%, 32%, 57% and 8%.

The realgar is selected, and a precipitate is taken after the water grind, and then the precipitate is dried and grinded for later use.

The radix pseudostellariae is first pulverized into a fine powder; then, the fine powder is crushed to a very fine powder and sterilized for later use.

The special grade indigo naturalis is selected, cleaned, refined, dried and sterilized for later use.

The fine powders of the above three pharmaceutical compositions are mixed for later use.
adding water to the salvia miltiorrhiza in accordance with liquid-to-solid ratio of 9ml/1g to obtain a solution , decocting the solution 2 times, for 55 min each time, then filtering the solution to obtain a filtrate, the filtrate is then combined and concentrated to get a clear paste with a relative density of 1.15 to 1.24 (50° C) at a temperature of 48-54 ° C.

The homogenized fine powders of the above three pharmaceutical compositions were added to the clear paste, granulated, dried, sized, and filled into a capsule (weight 0.25 g) to obtain the pharmaceutical composition for treating leukemia in a capsule form.

Different dosage forms of the pharmaceutical compositions for treating leukemia prepared in Embodiments 1-5 were used for clinical validation, and the compound realgar natural indigo tablet prepared by the embodiment of application No. 200410050453.1 was used as the control group. A total of 600 patients were selected and 100 patients were randomly assigned for each group. Taking the patient's physique and tolerance into account, the test group took a step-by-step approach to increase the dose. On the first day of treatment, 3 to 5 tablets or pills or tablets were used, 3 times a day, and about 10 days, 30 tablets or pills or tablets were taken per day, and 10 for each time. The control group was taken according to the administration method in the comparative document.

Efficacy criteria (cf. Suzhou National Leukemia Chemotherapy Symposium, 1987):
Complete Remission (CR): No clinical symptoms or signs due to leukemia cell infiltration, normal or nearly normal life, hemogram: Hb ≥ 100g/L (male) or ≥90g/L (female), neutrophil absolute valued ≥ 1.5 × 10⁹ / L, platelet ≥ 100 × 10⁹ / L, peripheral blood classification without leukemia cells, myelogram: the myeloblast + promyelocyte ≤ 5%, red blood cells and megakaryocytes are normal;
Partial remission (PR): myeloblasts of bone marrow + prom yelocytes of bone marrow >5% and myeloblasts of bone marrow + prom yelocytes of bone marrow ≤20%, or one of the clinical and hemogram did not reach the standard of complete remission.

Complete remission rate = numbers of complete remission per group / total numbers of patient per group; partial remission rate = 1 - complete remission rate. The specific results are shown in Table 1.

**Table 1 Effect after taking medicine**

| Groups | Complete remission rate (%) | Partial remission rate (%) |
|---|---|---|
| Embodiment 1 | 92.9% | 7.1% |
| Embodiment 2 | 93.0% | 7% |
| Embodiment 3 | 93.8% | 6.2% |
| Embodiment 4 | 94.1% | 5.9% |
| Embodiment 5 | 93.6% | 6.4% |
| Control Group | 82.1% | 17.9% |

As can be seen from Table 1, the therapeutic effect of the pharmaceutical composition for treating leukemia provided by the present disclosure is significantly higher than that of the control group; moreover, the pharmaceutical composition for treating leukemia provided by the present disclosure has small side effects and is more safe and effective.

## Claims

1. A pharmaceutical composition for treating leukemia, mainly made of the following raw materials by weight percentage comprising, 2 to 8 percent of realgar, 25 to 42 percent of indigo naturalis, 50 to 60 percent of salvia miltiorrhiza, and 6 to 10 percent of radix pseudostellariae.

2. The pharmaceutical composition for treating leukemia according to claim 1, wherein the raw materials by weight percentage comprising, 3 to 5 percent of realgar, 27 to 35 percent of indigo naturalis, 52 to 57 percent of salvia miltiorrhiza, and 7 to 9 percent of radix pseudostellariae.

3. The pharmaceutical composition for treating leukemia according to claim 1 or claim 2, wherein the indigo naturalis is excellent grade indigo naturalis or special grade indigo naturalis.

4. A method for preparing the pharmaceutical composition for treating leukemia according to any one of claims 1 to 3, comprising following steps:
mixing a realgar powder, a radix pseudostellariae powder and an indigo naturalis powder to obtain a mixture for standby application;
adding water to the salvia miltiorrhiza in accordance with liquid-to-solid ratio of 8ml/1g-9ml/1g to obtain a solution, decocting the solution 1-2 times, for 50-55 min each time, then filtering the solution to obtain a filtrate, the filtrate is then concentrated to get a concentrate at a temperature of 48-54 °C;
adding the mixture to the concentrate, and then mixing the mixture and the concentrate, making a corresponding pharmaceutical dosage form as required.

5. The method according to claim 4, wherein specific steps of preparing the realgar into a powder form are as follows: the realgar is selected, and a precipitate is taken after water grind, and then the precipitate is dried and grinded for later use.

6. The method according to claim 4, wherein specific steps of preparing the radix pseudostellariae into a powder form are: pulverizing the radix pseudostellariae into a fine powder; then, the fine powder is crushed to a very fine powder for later use.

7. The method according to any one of claims 4 to 6, wherein the pharmaceutical dosage form is tablets, pills, capsules, or granules.

8. The method according to claim 7, wherein the pharmaceutical dosage form is tablets;
the concentrate is a clear paste with a relative density of 1.15 to 1.20 measured at a temperature of 50°C; and then the clear paste is mixed with the mixture, granulated, dried, sized, compressed, and coated to obtain the tablets.

9. The method according to claim 7, wherein the pharmaceutical dosage form is pills; the concentrate is concentrated to a thick paste, then the thick paste is dried under reduced pressure into a dry paste, the dry paste is crushed into a fine powder to obtain a dry powder fine powder;
the dry powder fine powder is mixed with the mixture, and placed in a pill making machine with water and then dried to obtain the pills.

10. The method according to claim 7, wherein the pharmaceutical dosage form is capsules;
the concentrate is a clear paste with a relative density of 1.15 to 1.20 measured at a temperature of 50°C; and then the clear paste is mixed with the mixture, granulated, dried, sized, compressed, and filled into capsules.

## Patentansprüche

1. Pharmazeutische Zusammensetzung zur Behandlung von Leukämie, die hauptsächlich aus den folgenden Rohstoffen hergestellt wird, die nach Gewichtsprozent 2 bis 8 Prozent Realgar, 25 bis 42 Prozent Indigo naturalis, 50 bis 60 Prozent Salvia miltiorrhiza und 6 bis 10 Prozent Radix pseudostellariae umfassen.

2. Pharmazeutische Zusammensetzung zur Behandlung von Leukämie nach Anspruch 1, wobei die Rohstoffe nach Gewichtsprozent 3 bis 5 Prozent Realgar, 27 bis 35 Prozent Indigo naturalis, 52 bis 57 Prozent Salvia miltiorrhiza und 7 bis 9 Prozent Radix pseudostellariae umfassen.

3. Pharmazeutische Zusammensetzung zur Behandlung von Leukämie nach Anspruch 1 oder Anspruch 2, wobei es sich bei dem Indigo naturalis um Indigo naturalis von hervorragender Qualität oder Indigo naturalis von spezieller Qualität handelt.

4. Verfahren zur Herstellung der pharmazeutischen Zusammensetzung zur Behandlung von Leukämie nach einem der Ansprüche 1 bis 3, umfassend folgende Schritte:
Mischen eines Realgarpulvers, eines Radix pseudostellariae-Pulvers und eines Indigo naturalis-Pulvers, so dass eine Mischung für die Bereitschafts-Anwendung erhalten wird;
Zugeben von Wasser zu der Salvia miltiorrhiza gemäß einem Flüssigkeits-Feststoff-Verhältnis von 8 ml/1g-9 ml/1g, so dass eine Lösung erhalten wird, Abkochen der Lösung 1-2 mal für jeweils 50-55 min, dann Filtrieren der Lösung, so dass ein Filtrat erhalten wird, danach Konzentrieren des Filtrats, so dass ein Konzentrat bei einer Temperatur von 48-54°C erhalten wird;
Zugeben der Mischung zu dem Konzentrat und anschließendes Mischen der Mischung und des Konzentrats, Herstellen einer entsprechenden pharmazeutischen Dosierungsform wie gefordert.

5. Verfahren nach Anspruch 4, wobei spezifische Schritte zum Zubereiten des Realgars zu einer Pulverform wie folgt sind: der Realgar wird ausgewählt und ein Niederschlag wird nach dem Mahlen mit Wasser entnommen, und dann wird der Niederschlag getrocknet und zur späteren Verwendung gemahlen.

6. Verfahren nach Anspruch 4, wobei spezifische Schritte zum Zubereiten der Radix pseudostellariae zu einer Pulverform sind: Pulverisieren der Radix pseudostellariae zu einem feinen Pulver; dann wird das feine Pulver zur späteren Verwendung zu einem sehr feinen Pulver zerkleinert.

7. Verfahren nach einem der Ansprüche 4 bis 6, wobei es sich bei der pharmazeutischen Dosierungsform um Tabletten, Pillen, Kapseln oder Granulate handelt.

8. Verfahren nach Anspruch 7, wobei es sich bei der pharmazeutischen Dosierungsform um Tabletten handelt; das Konzentrat ist eine klare Paste mit einer relativen Dichte von 1,15 bis 1,20, gemessen bei einer Temperatur von 50°C; und dann wird die klare Paste mit der Mischung gemischt, granuliert, getrocknet, sortiert, gepresst und überzogen, so dass die Tabletten erhalten werden.

9. Verfahren nach Anspruch 7, wobei es sich bei der pharmazeutischen Dosierungsform um Pillen handelt; das Konzentrat wird zu einer dicken Paste konzentriert, dann wird die dicke Paste unter vermindertem Druck zu einer trockenen Paste getrocknet, die trockene Paste wird zu einem feinen Pulver zerkleinert, so dass ein trockenes Feinpulver erhalten wird, das trockene Feinpulver wird mit der Mischung gemischt und in eine Pillenherstellungsmaschine mit Wasser gegeben und dann getrocknet, so dass die Pillen erhalten werden.

10. Verfahren nach Anspruch 7, wobei es sich bei der pharmazeutischen Dosierungsform um Kapseln handelt; das Konzentrat ist eine klare Paste mit einer relativen Dichte von 1,15 bis 1,20, gemessen bei einer Temperatur von 50°C; und dann wird die klare Paste mit der Mischung gemischt, granuliert, getrocknet, sortiert, gepresst und in Kapseln gefüllt.

## Revendications

1. Composition pharmaceutique pour un traitement d'une leucémie, principalement composée des matières premières suivantes en pourcentage en poids, comprenant, 2 à 8 pour cent de réalgar, 25 à 42 pour cent d'indigo naturalis, 50 à 60 pour cent de salvia miltiorrhiza, et 6 à 10 pour cent de radix pseudostellariae.

2. Composition pharmaceutique pour un traitement de la leucémie selon la revendication 1, les matières premières comprenant, en pourcentage en poids, 3 à 5 pour cent de réalgar, 27 à 35 pour cent d'indigo naturalis, 52 à 57 pour cent de salvia miltiorrhiza, et 7 à 9 pour cent de radix pseudostellariae.

3. Composition pharmaceutique pour un traitement de la leucémie selon la revendication 1 ou la revendication 2, l'indigo naturalis étant de l'indigo naturalis de qualité excellente ou de l'indigo naturalis de qualité spéciale.

4. Procédé pour la préparation de la composition pharmaceutique pour un traitement d'une leucémie selon l'une quelconque des revendications 1 à 3, comprenant les étapes suivantes :
mélange d'une poudre de réalgar, d'une poudre de radix pseudostellariae et d'une poudre d'indigo naturalis pour obtenir un mélange pour une application de réserve ;
ajout d'eau à la salvia miltiorrhiza selon un rapport liquide-à-solide de 8 ml/1 g à 9 ml/1 g pour obtenir une solution, décoction de la solution 1 à 2 fois, pendant 50 à 55 min à chaque fois, ensuite filtration de la solution pour obtenir un filtrat, le filtrat étant ensuite concentré pour obtenir un concentré à une température de 48 à 54 °C ;
ajout du mélange au concentré, et ensuite mélange du mélange et du concentré,
préparation d'une forme de dosage pharmaceutique correspondante, au besoin.

5. Procédé selon la revendication 4, les étapes spécifiques de préparation du réalgar sous forme de poudre sont comme suit : le réalgar est choisi, et un précipité est pris après meulage à l'eau, et ensuite le précipité est séché et meulé pour une utilisation ultérieure.

6. Procédé selon la revendication 4, les étapes spécifiques de préparation du radix pseudostellariae sous forme de poudre sont comme suit : pulvérisation du radix pseudostellariae en une poudre fine ; ensuite, la poudre fine est broyée en une poudre très fine pour une utilisation ultérieure.

7. Procédé selon l'une quelconque des revendications 4 à 6, la forme de dosage pharmaceutique étant des comprimés, des pilules, des capsules, ou des granules.

8. Procédé selon la revendication 7, la forme de dosage pharmaceutique étant des comprimés ; le concentré étant une pâte transparente dotée d'une densité relative de 1,15 à 1,20 mesurée à une température de 50 °C ; et ensuite la pâte transparente est mélangée avec le mélange, granulée, séchée, dimensionnée, comprimée, et revêtue pour obtenir les comprimés.

9. Procédé selon la revendication 7, la forme de dosage pharmaceutique étant des pilules ; le concentré étant concentré en une pâte épaisse, ensuite la pâte épaisse étant séchée sous pression réduite en une pâte sèche, la pâte sèche étant broyée en une poudre fine pour obtenir une poudre fine de poudre sèche ;
la poudre fine de poudre sèche étant mélangée avec le mélange, et placée dans une machine pour fabriquer des pilules avec de l'eau et ensuite séchée pour obtenir les pilules.

10. Procédé selon la revendication 7, la forme de dosage pharmaceutique étant des capsules ; le concentré étant une pâte transparente dotée d'une densité relative de 1,15 à 1,20 mesurée à une température de 50 °C ; et ensuite la pâte transparente est mélangée avec le mélange, granulée, séchée, dimensionnée, comprimée, et remplie dans des capsules.
